# EUROPÄISCHE PATENTSCHRIFT

(11) **EP 2 228 366 B1**
(45) Veröffentlichungstag und Bekanntmachung des Hinweises auf die Patenterteilung: **28.12.2011**
(21) Anmeldenummer: 10002462.9
(22) Anmeldetag: 09.03.2010
(51) Int. Cl.: C07D 213/74

(54) **Verfahren zur Herstellung von 2-Amino-4-(halogenalkyl)pyridin-Derivaten durch Cyclisierung geeigneter Nitril-Vorstufen mit Stickstoff-Verbindungen**
Method for producing 2-amino-4-(halogenalkyl)pyridine derivatives by means of cyclization of suitable nitrile precursors with nitrogen combinations
Procédé de fabrication de dérivés de 2-amino-4-(halogène-alkyl)pyridine par fermeture du cycle de précurseurs de nitrile adaptés avec des liaisons d'azote

(30) Priorität: 12.03.2009 DE 102009012471; 07.04.2009 DE 102009016374
(43) Veröffentlichungstag der Anmeldung: 15.09.2010
(73) Patentinhaber: Archimica GmbH, 65929 Frankfurt am Main (DE)
(72) Erfinder: Jung, Jörg, Dr., 65439 Flörsheim (DE); Würtz, Sebastian, Dr., 65555 Limburg (DE)
(74) Vertreter: Schweitzer, Klaus

(56) Entgegenhaltungen:
- EP-A1- 0 228 846
- WO-A1-2007/000249
- MITTELBACH ET AL: "Synthesen mit Nitrilen, LXXV Zur Reaktivität der Dimeren von Malononitril und Cyanessigester gegenüber Dimethylformamid-dimethylacetal" MONATSHEFTE FÜR CHEMIE, Bd. 118, 1987, Seiten 617-626, XP002587682 ISSN: 0026-9247 DOI: 10.1007/BF00809673

## Beschreibung

Das erfindungsgemäße Verfahren betrifft die Umsetzung geeigneter Nitril-Vorstufen mit Stickstoffverbindungen zu den gewünschten substituierten Pyridinen. Die Herstellung der Ausgangprodukte erfolgt dabei im allgemeinen durch Addition eines alpha-metallierten Nitrils an eine Carbonylverbindung.

Pyridine sind wichtige Strukturelemente in einer Vielzahl von Produkten der chemischen und pharmazeutischen Industrie und es sind in der Literatur sehr viele verschiedene Verfahren zur Herstellung beschrieben. Diese können grob unterteilt werden in Verfahren, bei denen der Pyridinring aufgebaut wird und solche, bei denen Substituenten eingeführt (z. B. durch elektrophile oder nucleophile Substituion am Aromaten) oder modifiziert werden.

Werden 2-Aminopyridinderivate benötigt, so erfolgt die Herstellung meistens durch Einführung des Amin-Substituenten in einen bereits vorhandenen Pyridinring. Beispiele für solche Reaktionen sind die Tschitschibabin-Reaktion (siehe z. B. DE-B-37 42 91), also die Umsetzung von Pyridinen mit Natriumamid unter Eliminierung von Natriumhydrid oder die Umsetzung von 2-Halogenpyridinen mit Stickstoffverbindungen (siehe z. B. Chem. Ber. 1936, 69, 2593 für die Umsetzung von 3-Amino-2-chlorpyridin zu 2,3-Diaminopyridin).

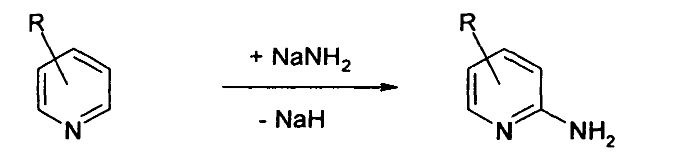

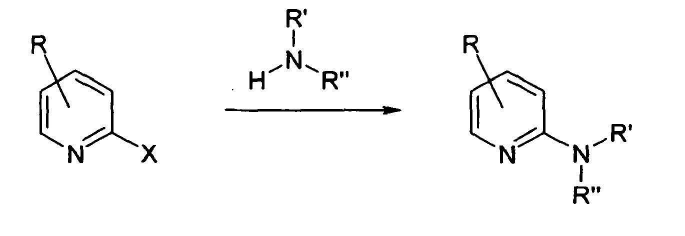

Diese Reaktionen haben jedoch den Nachteil, daß entweder recht drastische Bedingungen nötig sind (Tschitschibabin-Reaktion, Reaktion von 2-Halogenpyridinen mit wäßrigem Ammoniak) oder daß bei den neueren katalytischen Varianten zur Umsetzung teure Edelmetalle und Liganden benötigt werden (z. B. Org. Lett. 2001, 3, 3417).

Ein weiterer Nachteil der beschriebenen Reaktionen ist die Tatsache, daß zur Synthese von schwierig substituierten Pyridinen die entsprechenden Vorstufen verfügbar sein müssen, was oft nicht der Fall ist. Außerdem muß für das Einführen des gewünschten Substituenten in die gewünschte Position ein technisch durchführbares Verfahren zur Verfügung stehen, das die Umwandlung der Vorstufe auch jenseits des Labormaßstabs erlaubt.

Dies ist insbesondere oft dann nicht der Fall, wenn Perfluoralkylgruppen (meist Trifluormethylgruppen) in den Pyridin-Ring eingeführt werden sollen. Zwar sind hier einige Reaktionen in der Literatur beschrieben, wie die Umsetzung von lodpyridinen mit (Trifluormethyl)trimethylsilan 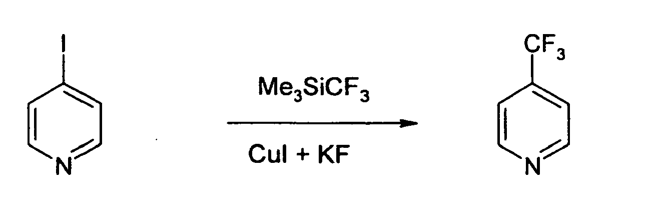 oder die Umwandlung von Methylgruppen in Trifluormethylgruppen durch Einwirkung von Chlor und Flußsäure.

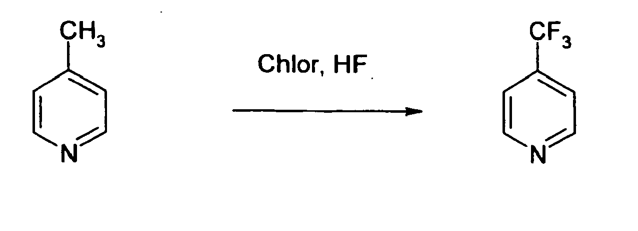

Alle bisher bekannten Verfahren haben aber Nachteile, die die Verwendung für die Herstellung der gesuchten Aminopyridine unattraktiv macht bzw. ausschließt. So sind die Aminopyridine unter den drastischen Bedingungen der Umwandlung von Methyl- in Trifluormethylgruppen nicht stabil. Es müßten also zunächst andere Pyridinderivate, z. B. Halogenpyridine herstellt werden und in einem gesonderten Schritt die Umwandlung von Halogen in Amin durchgeführt werden, was in aufwendigen und teueren Verfahren resultiert. Die Einführung einer Trifluormethylgruppe durch Umwandlung eines lodpyridins mit Hilfe von (Trifluormethyl)trimethylsilan ist für den technischen Maßstab wegen der hohen Preise der Startmaterialien ebenfall kaum attraktiv.

Aufgabe der vorliegenden Erfindung war es demnach ein Verfahren zur Verfügung zu stellen, mit dessen Hilfe die gewünschten 2-Aminopyridin-Derivate mit hoher Flexibilität bezüglich des Substitutionsmusters hergestellt werden können und mit dem insbesondere perfluoralkylsubstituierte, bevorzugt trifluormethylsubstituierte 2-Aminopyridine hergestellt werden können.

Ein ähnliches Verfahren wurde bereits für 2-Halogenpyridine beschrieben (WO2007/000249). Bei diesem Verfahren wird ein Nitril zunächst metalliert und dann mit einer geeigneten Carbonylverbindung zum Hydroxynitril umgesetzt. Der finale Ringschluß erfolgt dann unter stark sauren Bedingungen mit HX (HCl, HBr, HI) oder anorganischen Estern dieser Substanzen (z. B. SOCl₂, POCl₃, PCl₅, PBr₃ etc.) unter sehr stark sauren Bedingungen. Beispielhaft ist diese Reaktion für die Synthese von 4-Trifluormethyl-2-chlorpyridin erläutert.

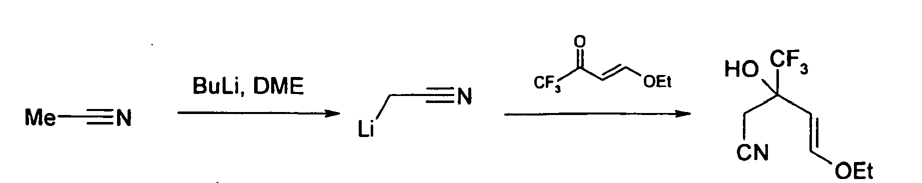

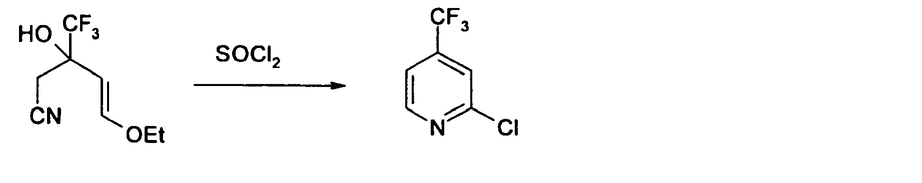

Um hiervon ausgehend das entsprechende 2-Aminopyridin herstellen zu können, ist jedoch eine weitere Umsetzung mit Ammoniak nötig, die nur unter drastischen Bedingungen abläuft (siehe EP-B-0 228 846 oder Dunn et al. in J. Fluor. Chem 1999, Seite 153) und hohe Temperaturen und hohen Druck erfordert.

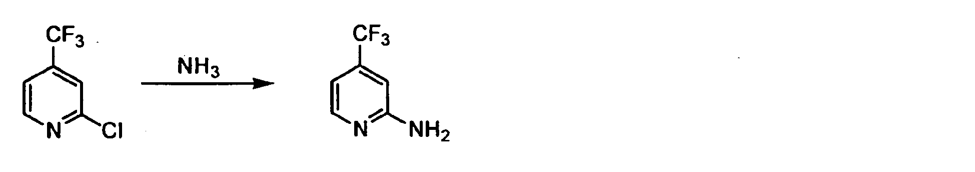

Vor diesem Hintergrund bestand die Aufgabe darin, ein ökonomisches und technisch einfach durchführbares Verfahren für die Herstellung von 2-Amino-4-(fluoralkyl)pyridin-Derivaten zu entwickeln.

Es wurde nun gefunden, daß sich diese Aufgabe durch direkte Umsetzung der in W02007/000249 beschriebenen Nitrilvorstufen mit Ammoniak oder anderen geeigneten Stickstoffverbindungen lösen läßt.

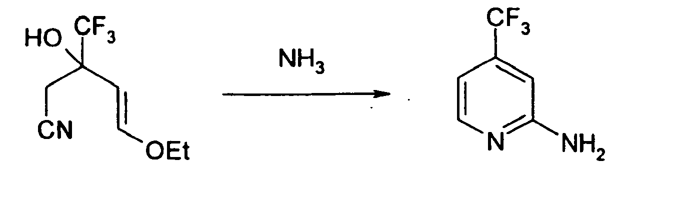

Dies ist überraschend, weil das Arbeiten unter stark sauren Bedingungen in der oben beschriebenen Umsetzung zum Halogenpyridin als entscheidend für den Erfolg angesehen wurde.

Es resultiert so ein allgemeines und flexibles Verfahren, mit dem substituierte 2-Aminopyridine, insbesondere 2-Aminopyridine mit Halogen substituierten Alkylsubstituenten durch Aufbau des Pyridinringes hergestellt werden können:

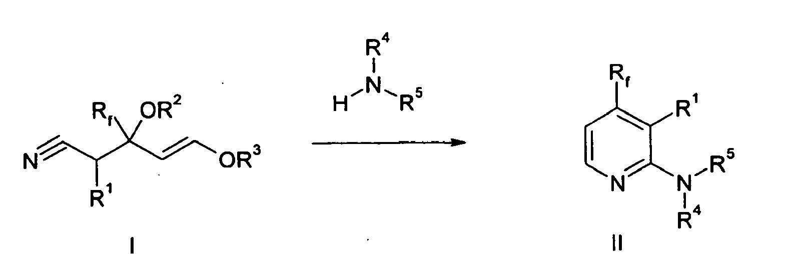

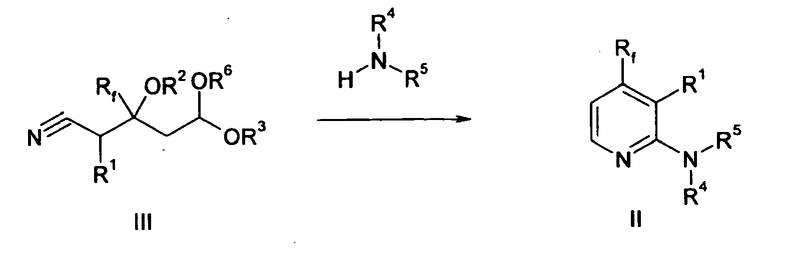

Bei den verschiedenen Resten im obigen Schema handelt es sich um folgende Substituenten:
- R_{f}:: CnH₍₂ₙ₊₁₋ₘ₎Xₘ
- X:: F, Cl, Br, wobei für m>1 X identisch oder verschieden ist
- n:: positive ganze Zahl zwischen 1 und 8
- m:: positive ganze Zahl zwischen 1 und 2n+1
- R¹:: Wasserstoff, C1-C8-Alkyl, C6-C14-Aryl, C1-C13-Heteroaryl, COOR, CN, SO₂R, SOR, PO(OR)₂, wobei diese Alkyl-, Aryl-, Heteroaryl-, - COOR, -CN, -SO₂R, -SOR und -PO(OR)₂-Reste unsubstituiert oder ein- oder mehrfach substituiert sind,
- R²:: Wasserstoff oder eine Schutzgruppe für Alkohole
- R³, R⁶:: C1-C8-Alkyl, C1-C8-Acyl, C6-C14-Aryl, R₃Silyl, wobei die Alkyl-, Acyl- und -Aryl-Reste unsubstituiert oder ein- oder mehrfach substituiert sind,
- R⁴, R⁵:: Wasserstoff, C1-C8-Alkyl, C6-C14-Aryl, C1-C13-Heteroaryl, C1-C8- Acyl, -CONR₂, wobei diese Alkyl-, Aryl-, Heteroaryl-, Acyl- und - CONR2-Reste unsubstituiert oder ein- oder mehrfach substituiert sind, oder R⁴ und R⁵ bilden zusammen eine C4-C8-Alkylenkette, wobei auch eine CH₂-Gruppe durch NH oder O ersetzt sein kann und wobei diese C4-C8-Kette unsubstituiert oder ein- oder mehrfach substituiert ist
- R:: C1-C8-Alkyl, C6-C14-Aryl, C1-C13-Heteroaryl

Unter "Alkyl" im Sinne der Erfindung wird - sofern nicht anders angegeben - ein verzweigter oder unverzweigter C₁- bis C₂₀-, bevorzugt ein C₁- bis C₁₀-Alkylrest verstanden, insbesondere Methyl, Ethyl, Propyl, Isopropyl, Butyl, Isobutyl, Tertiärbutyl, Pentyl oder Hexyl, besonders bevorzugt Methyl, Ethyl, Propyl oder Butyl.

Sofern nicht anders angegeben, handelt es sich bei "Aryl" (oder einem "aromatischen" Rest) um einen C₆- bis C₁₄-Arylrest, insbesondere um Phenyl, Naphthyl, Diphenyl. Diese aromatischen Substituenten können dabei ihrerseits zusätzlich Alkyl- oder andere Aryl-Substitutenten tragen.

Sofern nicht anders angegeben, handelt es sich bei "Heteroaryl" (oder einem "heteroaromatischen" Rest) um einen C₅- bis C₁₃-Arylrest, worin 1, 2 oder mehr Kohlenstoffatome unabhängig voneinander durch O, S, N, N-Alkyl ersetzt sein können, insbesondere um Furyl, Thiophenyl, Oxazolyl, Thiazolyl, Pyridyl, Pyrimidyl, Indolyl, Chinoxalinyl oder Pyrrolyl.

Unter "Halogen" oder "Halogenid" wird, sofern nichts anderes angegeben ist, ein Fluor-, Chlor-, Brom- oder lodrest verstanden, vorzugsweise ein Brom- oder Chlorrest.

Typische "Schutzgruppen für Alkohole" sind z. B. Acyl, Alkyl, 2-tetrahydropyranyl oder R₃Silyl (mit R= Methyl, Ethyl, Propyl, tert-Butyl). Die jeweiligen Reste R können dabei entweder alle gleich oder auch unterschiedlich sein.

"Ein- oder mehrfach substituiert" oder "substituiert" im Sinne der Erfindung bedeutet, dass der jeweilige Rest ein- oder sofern möglich, zwei-, drei- oder mehrfach durch Halogen, bevorzugt Chlor oder Brom, und/oder durch Nitro, Cyano, Hydroxy, Alkyl, Alkyloxy, Aryl, Aryloxy, Arylalkyl, Aryloxyalkyl, Arylalkyloxy, Amino, Monoalkylamino, Dialkylamino, Monoarylamino, Diarylamino, Alkylthio, Arylthio, Carboxy, Alkylcarbonyl und/oder Alkyloxycarbonyl substituiert ist.

Die Herstellung der benötigten Nitrilvorstufen kann dabei nach dem Fachmann geläufigen Verfahren erfolgen. Meist dürfte aber die Herstellung nach einem der beiden folgenden Schemata ausgehend vom ungesättigten Keton IV oder vom Acetal V besonders ökonomisch sein:

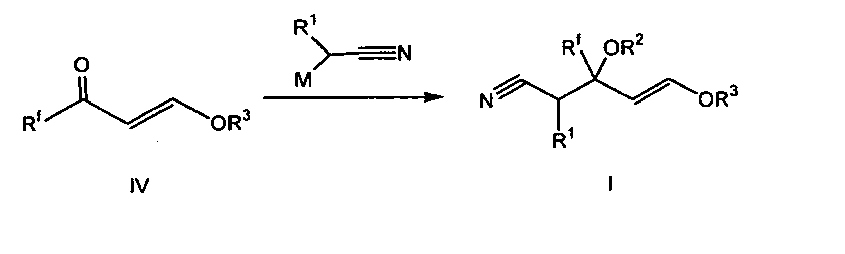

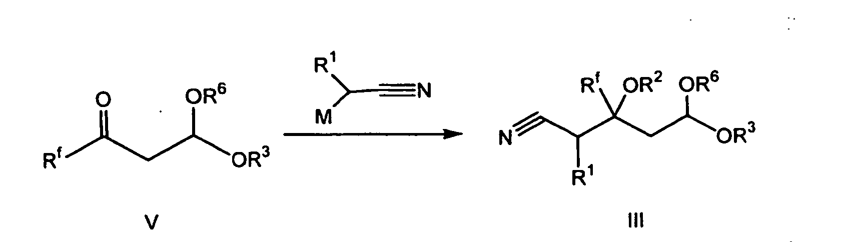

Die Bedeutung der Reste R ist dabei die gleiche, wie oben erläutert. Zusätzlich steht M für die folgenden Metalle:
- M:: Li, Na, K, MgY, Mg_{0,5}, CaY, Ca_{0,5}, ZnY, Zn_{0,5}, CdY, Cd_{0,5}, Cu, TiY₃
- Y:: X (wie oben definiert), I, OR, O-CO-R (mit R wie oben definiert)

Zur Synthese der zur Cyclisierung benötigten Nitrile ist, wie oben in den Formelschemata beschrieben, im allgemeinen der Zugang aus den Ketonen IV oder V und einem Salz eines Acetonitril-Derivates der günstigste Weg. Dazu wird zunächst Acetonitril oder ein substituiertes Derivat in einem geeigneten Lösungsmittel metalliert und das entstandene Salz dann mit einem Keton der allgemeinen Formel IV oder V umgesetzt.

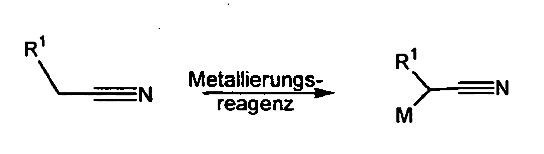

Für diese Reaktion sind alle Lösungsmittel geeignet, die für Metallierungsreaktionen eingesetzt werden können. Dies sind insbesondere Ether wie Tetrahydrofuran, 2-Methyltetrahydrofuran, Diethylether, Diisopropylether, Di-n-butylether, Dioxan, 1,2-Dimethoxyethan, Diethylengylcoldimethylether, Diethylenglcoldi-n-butylether, Tetraethylenglycoldimethylether oder Mischungen dieser Lösungsmittel untereinander oder mit einem inerten anderen Lösungsmittel wie Benzol, Toluol, Xylol, Cyclohexan oder Petrolethern (Kohlenwasserstoffgemische). In besonderen Fällen können aber auch reine Kohlenwasserstoffe wie Benzol, Toluol, Xylol, Cyclohexan oder Petrolether geeignet sein oder im Falle von stark aciden Acetonitril-Derivaten (R' starker Akzeptor-Substituent) sogar Alkohole wie Methanol, Ethanol, Isopropanol oder Butanole.

Als Metallierungsreagenzien kommen alle Basen in Frage, die ausreichend basisch sind, um ein Wasserstoffatom von dem gegebenenfalls substituierten Acetonitril zu abstrahieren. Bei Acetonitril selbst oder Alkyl-substituierten Acetonitrilen kommen dafür hauptsächlich sehr starke Basen wie n-Butyllithium, sec-Butyllithium, t-Butyllithium, n-Hexyllithium, Lithium-N,N-diisopropylamid (LDA), Lithium-2,2,6,6-tetramethylpiperidid (Li-TMP), Lithiumhexamethyldisilazan (LiHMDS), Natriumhexamethyldisilazan (NaHMDS) oder Kaliumhexamethyldisilazan (KHMDS) in Frage. Bei etwas acideren Acetonitril-Derviaten wie beispielsweise Arylsubstituierten (R' = Aryl) sind Basen wie Natriumamid, Lithiumhydrid, Natriumhydrid oder Kaliumhydrid zusätzlich zu den oben genannten geeignet. Bei den am stärksten aciden Acetonitril-Derivaten (R' = COOR, CN, SO₂R, SOR, PO(OR)₂) sind zusätzlich zu den bereits genannten starken Basen auch Alkoxide wie die Lithium- Natrium-, oder Kaliumsalze von Methanol, Ethanol oder t-Butanol als Basen geeignet.

Die Reaktionsbedingungen, die bei der Metallierung zweckmäßigerweise einzuhalten sind, hängen wiederum von den verwendeten Acetonitril-Derivaten ab. So wird bei den am wenigsten aciden Acetonitril-Derivaten (R¹ = Alkyl oder Wasserstoff) bevorzugt bei Temperaturen unter - 25 °C gearbeitet und besonders bevorzugt unter -45 °C, um die Zersetzung der gebildeten Salze zu vermeiden. Die acideren Acetonitril-Derivate können wegen der größeren Stabilität der gebildeten Salze auch bei höheren Temperaturen metalliert werden (für R¹ = Aryl bis zu ca. 0 °C; für R¹ = CN, COOR, SO₂R, SOR auch bei Raumtemperatur oder sogar darüber).

Die sich anschließende Umsetzung mit geeigneten Ketonen mit den allgemeinen Formeln IV oder V wird am besten bei der gleichen Temperatur durchgeführt wie die Metallierung und erfolgt im allgemeinen durch Zugabe der Ketone zum metallierten Acetonitril oder Acetonitril-Derivat. Die Zugabe-Reihenfolge kann jedoch auch vertauscht sein. Die Aufarbeitung des Reaktionsgemisches schließlich erfolgt meist durch Neutralisieren der enthaltenen Base mit einer geeigneten Säure (z. B. Schwefelsäure, Essigsäure, Zitronensäure, Salzsäure) und Entfernen des gebildeten Salzes mit Wasser. Das so entstandene Produkt wird mit üblichen Techniken wie Destillation oder Kristallisation gereinigt oder kann oft auch roh in die Folgestufe eingesetzt werden. In manchen Fällen kann es auch vorteilhaft sein, nicht mit einer Protonenquelle zu quenchen, sondern mit anderen Elektrophilen. Es entstehen dann nicht die Alkohole (R² = H), sondern entsprechende Derivate (R² = Alkyl, Acyl, 2-Tetrahydropyranyl, R₃Silyl) als Ausgangsmaterialien für die Cyclisierung.

Die Cyclisierung der Vorstufen I oder III zu den gewünschten Aminopyridinderivaten II kann mit allen geeigneten Stickstoffverbindungen erfolgen, d. h. mit solchen, bei denen R⁴ und R⁵ wie oben angegeben unabhängig voneinander Wasserstoff, Alkyl, Aryl, Heteroaryl, Acyl, oder -CONR₂ sind. Beide Substituenten können dabei auch Teil eines Ringsystems sein. Die Cyclisierungsreaktion wird im allgemeinen in einem geeigneten Lösungsmittel durchgeführt. Dies ist im einfachsten Fall die Stickstoffverbindung selbst oder eine Mischung der Stickstoffverbindung mit anderen Lösemitteln oder Lösemittelgemischen. Geeignete Lösemittel sind alle, die die Reaktion nicht behindern also z. B. Ether (Dioxan, THF, MTBE, Diisopropylether, Din-butylether), Aromaten (Toluol, Xylol, Benzol, Chlorbenzol, Anisol), Alkohole (Methanol, Ethanol, Propanol, Isopropanol, Butanol) oder Wasser.

Die Reaktion wird durchgeführt durch einfaches Erwärmen der Vorstufen I oder III mit den Stickstoff-Verbindungen in einem optional zu verwendenden Lösemittel. Typische Temperaturen sind dabei 40 °C bis 250 °C, bevorzugt 60 °C bis 200 °C und besonders bevorzugt 80 °C bis 150 °C. Der Zusatz eines Katalysators ist zur Erzielung der Cyclisierung normalerweise nicht nötig, es können aber bei Bedarf saure oder alkalische Additive zugesetzt werden, um die Cyclisierungsreaktion zu beschleunigen. Als saure Additive werden bevorzugt Salze der verwendeten Stickstoffverbindungen eingesetzt, besonders bevorzugt Salze von Salzsäure, Schwefelsäure, Phosphorsäure, p-Toluolsulfonsäure, Essigsäure oder Zitronensäure. Als basische Additive eignen sich Hydroxide, Carbonate, Oxide, Alkoholate und andere stark basische Verbindungen, bevorzugt solche, die stärker basisch sind, als die verwendeten Stickstoffverbindungen.

Die Aufarbeitung erfolgt in Abhängigkeit von den Eigenschaften des Produktes durch Destillation oder Kristallisation.

### Beispiel 1: Herstellung von 5-Ethoxy-3-hydroxy-3-(trifluormethyl)-pent-4-ennitril als Cyclisierungsvorstufe

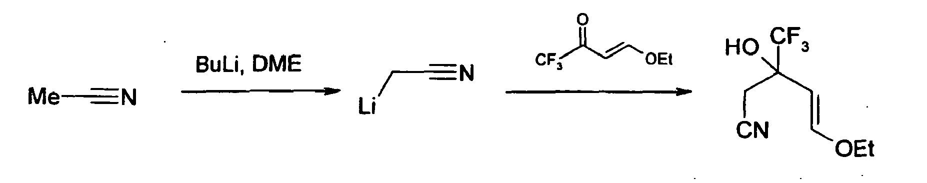

500 ml 1,2-Dimethoxyethan wurden auf -72 °C gekühlt und bei dieser Temperatur zunächst mit 126 ml n-BuLi (2,5 molar in Hexan) und dann innerhalb von 2 h ebenfalls bei -72 °C mit 12,8 g Acetonitril versetzt. Das Gemisch wurde nun 90 min nachrühren gelassen, um die Bildung des Anions zu vervollständigen. Anschließend wurde bei -72 °C innerhalb von 2 h mit einer Lösung von 50 g 1,1,1-Trifluoro-but-3-en-2-one (Herstellung gemäß Chem. Ber. 1989, 122, 1179 - 1186) in 100 ml 1,2-Dimethoxyethan versetzt und dann 1 h bei dieser Temperatur nachrühren gelassen. Anschließend wurde das Gemisch auf 0 °C erwärmt und zum Neutralisieren mit einer Lösung von 16,1 g Schwefelsäure (96%ig) in 50 ml Wasser versetzt.

Anschließend wurden 500 ml Toluol zugegeben, die Phasen getrennt und die wäßrige Phase zweimal mit weiteren 100 ml Toluol gegenextrahiert. Die vereinigten organischen Phasen wurden mit Natriumsulfat getrocknet und dann am Rotationsverdampfer konzentriert. Schließlich wurde das Produkt im vollen Ölpumpenvakuum (ca. 0,2 mbar) destilliert. Es konnten so 48,5 g Produkt (78 %) vom Siedepunkt 95 bis 110 °C gewonnen werden. Dieses wurde anhand seines Massenspektrums identifiziert (M+ = 209, weitere Fragmente bei m/e= 169, 141 und 71).

### Beispiel 2: Herstellung von 2-Amino-4-(trifluormethyl)pyridin aus 5-Ethoxy-3-hydroxy-3-(trifluormethyl)-pent-4-ennitril mit wäßrigem Ammoniak

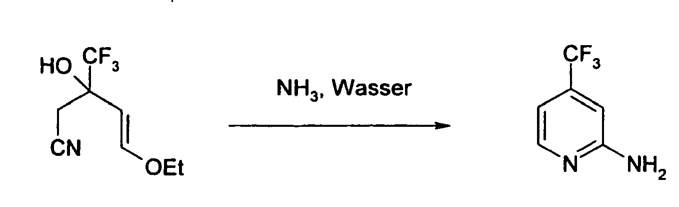

Es wurden 50 g 5-Ethoxy-3-hydroxy-3-(trifluormethyl)-pent-4-en-nitril mit 600 g wäßrigem Ammoniak (25%ig) gemischt und die resultierende Mischung wurde 24 h in einem Autoklaven auf 125 °C erhitzt, wobei sich ein Druck von ca. 14 bar aufbaute. Anschließend wurde das Reaktionsgemisch abgekühlt und das resultierende zweiphasige Gemisch mehrmals mit Dichlormethan extrahiert. Die vereinigten organischen Phasen wurden vorsichtig einrotiert und das Produkt anschließend aus Cyclohexan umkristallisiert. Es konnten so 26,3 g 2-Amino-4-(trifluormethyl)pyridin (68 %) als gelblichbrauner Feststoff gewonnen werden. Die spektroskopischen Daten stimmten mit den in der Literatur angegebenen überein (A. D. Dunn et al. in J. Fluorine Chem. 1999, 93, 153-157).

### Beispiel 3: Herstellung von Dimethyl-(4-trifluormethyl-pyridin-2-yl)-amin aus 5-Ethoxy-3-hydroxy-3-(trifluormethyl)-pent-4-ennitril mit wäßriger Dimethylamin-Lösung

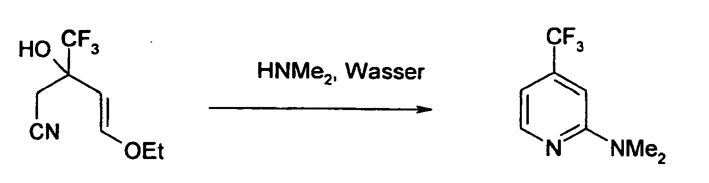

Es wurde analog zu Beispiel 2 gearbeitet, aber statt 600 g wäßrigem Ammoniak wurden 600 g wäßrige Dimethylamin-Lösung (40%ig) eingesetzt. Es konnten so 28,1 g (62 %) Produkt isoliert werden.

### Beispiel 4: Herstellung von 5-Ethoxy-3-hydroxy-2-phenyl-3-trifluormethyl-pent-4-enenitril als Cyclisierungsvorstufe

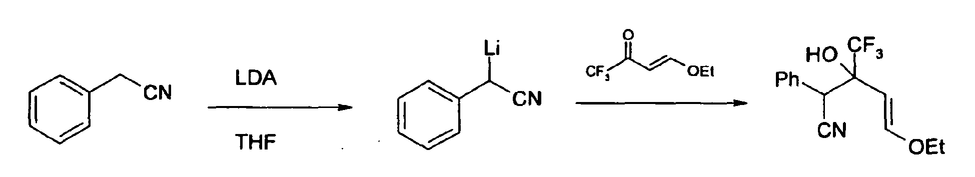

500 ml THF wurden auf -72 °C gekühlt und bei dieser Temperatur zunächst mit 31,9 g Diisopropylamin und dann bei derselben Temperatur mit 126 ml n-BuLi (2,5 molar in Hexan) versetzt. Anschließend wurden 35,1 g Benzylcyanid gelöst in weiteren 250 ml THF innerhalb von 1 h zugetropft. Es wurde weitere 2 h nachrühren gelassen, um die Bildung des Anions zu vervollständigen. Anschließend wurde bei -72 °C innerhalb von 2 h mit einer Lösung von 50 g 1,1,1-Trifluoro-but-3-en-2-one versetzt und dann 1 h bei dieser Temperatur nachrühren gelassen. Anschließend wurde das Gemisch auf 0 °C erwärmt und zum Neutralisieren mit einer Lösung von 16,1 g Schwefelsäure (96%ig) in 50 ml Wasser versetzt. Anschließend wurden 500 ml Toluol zugegeben, die Phasen getrennt und die wäßrige Phase zweimal mit weiteren 100 ml Toluol gegenextrahiert. Die vereinigten organischen Phasen wurden mit Natriumsulfat getrocknet und dann am Rotationsverdampfer konzentriert. Es wurden so ca. 77 g eines Rohproduktes gewonnen, das so in der Folgestufe eingesetzt wurde.

### Beispiel 5: Herstellung von 3-Phenyl-4-trifluormethyl-pyridin-2-ylamin durch Cyclisierung von 5-Ethoxy-3-hydroxy-2-phenyl-3-trifluormethyl-pent-4-enenitril mit Ammoniakwasser

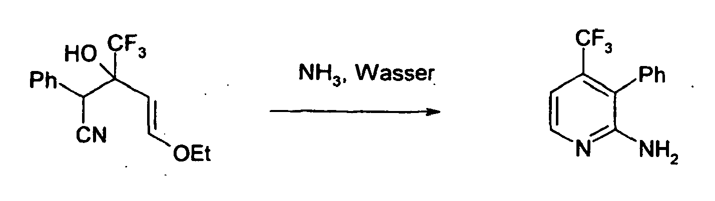

Es wurden 50 g 5-Ethoxy-3-hydroxy-2-phenyl-3-trifluoromethyl-pent-4-enenitrile mit 600 g wäßrigem Ammoniak (25%ig) gemischt und die resultierende Mischung wurde 24 h in einem Autoklaven auf 125 °C erhitzt. Anschließend wurde das Reaktionsgemisch abgekühlt und das resultierende zweiphasige Gemisch mehrmals mit Dichlormethan extrahiert. Die vereinigten organischen Phasen wurden einrotiert und das Produkt anschließend aus Heptan umkristallisiert. Es konnten so 24,1 g 3-Phenyl-4-trifluoromethyl-pyridin-2-ylamin (52 % über beide Stufen) isoliert werden.

## Patentansprüche

1. Verfahren zur Herstellung von 2-Aminopyridin-Derivat der allgemeinen Formel II, **dadurch gekennzeichnet, dass** eine offenkettige Nitril-Vorstufe I oder III mit einer Stickstoffverbindung in einer Cyclisierungsreaktion umgesetzt wird: 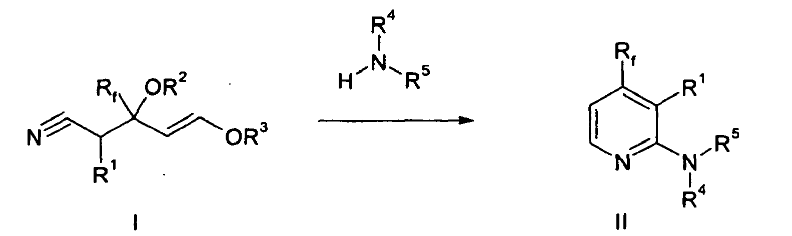 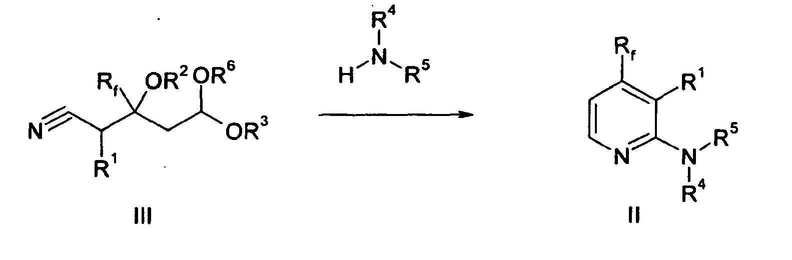 wobei die Substituenten R¹ bis R⁶ und R^{f} die folgenden Bedeutungen haben:
R_{f}: CnH(2ₙ₊₁₋ₘ)Xₘ
X: F, Cl, Br, wobei für m>1 X identisch oder verschieden ist
n: positive ganze Zahl zwischen 1 und 8
m: positive ganze Zahl zwischen 1 und 2n+1
R¹: Wasserstoff, C1-C8-Alkyl, C6-C14-Aryl, C1-C13-Heteroaryl, COOR, CN, SO₂R, SOR, PO(OR)₂, wobei diese Alkyl-, Aryl-, Heteroaryl-, - COOR, -CN, -SO₂R, -SOR und -PO(OR)₂-Reste unsubstituiert oder ein- oder mehrfach substituiert sind,
R²: Wasserstoff oder eine Schutzgruppe für Alkohole
R³, R⁶: C1-C8-Alkyl, C1-C8-Acyl, C6-C14-Aryl, R₃Silyl, wobei die Alkyl-, Acyl- und -Aryl-Reste unsubstituiert oder ein- oder mehrfach substituiert sind,
R⁴, R⁵: Wasserstoff, C1-C8-Alkyl, C6-C14-Aryl, C1-C13-Heteroaryl, C1-C8- Acyl, -CONR₂, wobei diese Alkyl-, Aryl-, Heteroaryl-, Acyl- und - CONR2-Reste unsubstituiert oder ein- oder mehrfach substituiert sind, oder R⁴ und R⁵ bilden zusammen eine C4-C8-Alkylenkette, wobei auch eine CH₂-Gruppe durch NH oder O ersetzt sein kann und wobei diese C4-C8-Kette unsubstituiert oder ein- oder mehrfach substituiert ist
R: C1-C8-Alkyl, C6-C14-Aryl, C1-C13-Heteroaryl.

2. Verfahren nach Anspruch 1, wobei die Cyclisierung ausgehend vom Enolether I durchgeführt wird.

3. Verfahren nach Anspruch 2, **dadurch gekennzeichnet, daß** der Enolether I ausgehend von Keton IV durch Umsetzung mit metalliertem Acetonitril oder einem metallierten Acetonitril-Derivat gemäß folgendem Reaktionsschema erhalten wird: 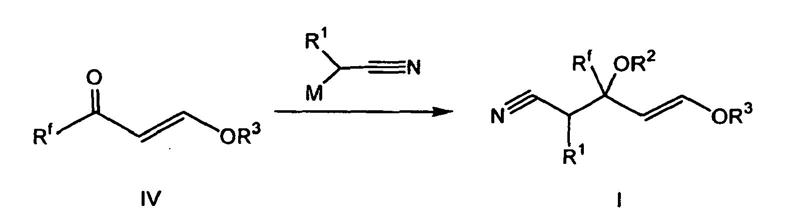 wobei
M für Li, Na, K, MgY, Mg_{0,5}, CaY, Ca_{0,5}, ZnY, Zn_{0,5}, CdY, Cd_{0,5}, Cu, TiY₃ und
Y für X oder I, OR, O-CO-R steht.

4. Verfahren nach Anspruch 1, bei dem die Cyclisierung ausgehend vom Acetal III durchgeführt wird.

5. Verfahren nach Anspruch 4, **dadurch gekennzeichnet, daß** Acetal III ausgehend von Keton V durch Umsetzung mit metalliertem Acetonitril oder einem metallierten Acetonitril-Derivat im Sinne des folgenden Reaktionsschemas umgesetzt wird. 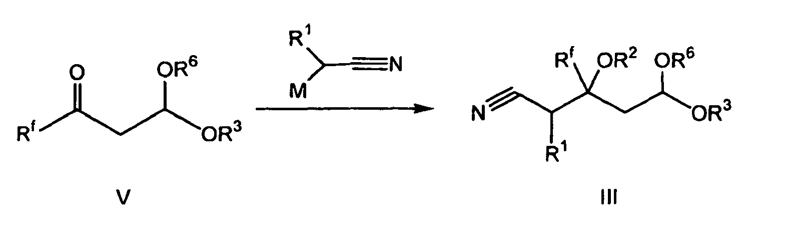 wobei
M für Li, Na, K, MgY, Mg_{0,5}, CaY, Ca_{0,5}, ZnY, Zn_{0,5}, CdY, Cd_{0,5}, Cu, TiY₃ und
Y für X oder I, OR, O-CO-R steht.

6. Verfahren nach einem der Ansprüche 1 bis 5, **dadurch gekennzeichnet, daß** R² = Wasserstoff ist.

7. Verfahren nach einem der Ansprüche 1 bis 6, **dadurch gekennzeichnet, daß** der Rest R_{f} gleich Trifluormethyl ist.

8. Verfahren nach einem der Ansprüche 1 bis 7, **dadurch gekennzeichnet, daß** es sich bei der Stickstoffverbindung um ein Amin handelt.

9. Verfahren nach einem der Ansprüche 1 bis 8, **dadurch gekennzeichnet, daß** es sich bei der Stickstoffverbindung um Ammoniak handelt.

## Claims

1. Process for preparing 2-aminopyridine derivative of the general formula II, **characterized in that** an open-chain nitrile precursor I or III is reacted with a nitrogen compound in a cyclization reaction: 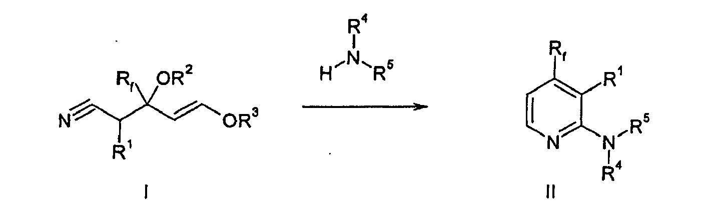 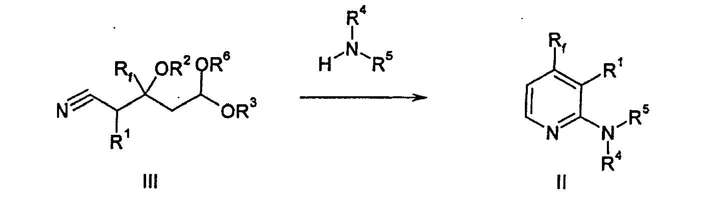 where the substituents R¹ to R⁶ and R^{f} are each defined as follows:
R_{f}: CₙH₍₂ₙ₊₁₋ₘ₎Xₘ
X: F, Cl, Br, where X is identical or different when m>1
n: positive integer from 1 to 8
m: positive integer from 1 to 2n+1
R¹: hydrogen, C1-C8-alkyl, C6-C14-aryl, C1-C13- heteroaryl, COOR, CN, SO₂R, SOR, PO(OR)₂, where these alkyl, aryl, heteroaryl, -COOR, -CN, -SO₂R, -SOR and -PO(OR)₂ radicals are unsubstituted or mono- or polysubstituted,
R²: hydrogen or a protecting group for alcohols
R³, R⁶: C1-C8-alkyl, C1-C8-acyl, C6-C14-aryl, R₃silyl, where the alkyl, acyl and aryl radicals are unsubstituted or mono- or poly-substituted,
R⁴,R⁵: hydrogen, C1-C8-alkyl, C6-C14-aryl, C1-C13- heteroaryl, C1-C8-acyl, -CONR₂ where these alkyl, aryl, heteroaryl, acyl and -CONR2 radicals are unsubstituted or mono- or poly- substituted, or R⁴ and R⁵ together form a C4-C8-alkylene chain, where one CH₂ group may also be replaced by NH or O and where this C4-C8 chain is unsubstituted or mono- or poly- substituted
R: C1-C8-alkyl, C6-C14-aryl, C1-C13-heteroaryl.

2. Process according to Claim 1, wherein the cyclization is performed proceeding from enol ether I.

3. Process according to Claim 2, **characterized in that** the enol ether I is obtained proceeding from ketone IV by reacting with metallated acetonitrile or a metallated acetonitrile derivative according to the following reaction scheme: 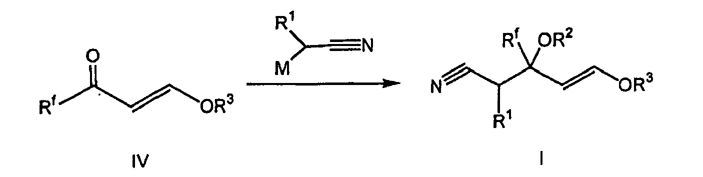 where
M is Li, Na, K, MgY, Mg_{0.5}, CaY, Ca_{0.5}, ZnY, Zn_{0.5}. CdY, Cd_{0.5}, Cu, TiY₃ and
Y is X or I, OR, O-CO-R.

4. Process according to Claim 1, in which the cyclization is performed proceeding from the acetal III.

5. Process according to Claim 4, **characterized in that** acetal III, proceeding from ketone V, is converted by reaction with metallated acetonitrile or a metallated acetonitrile derivative in the manner of the following reaction scheme: 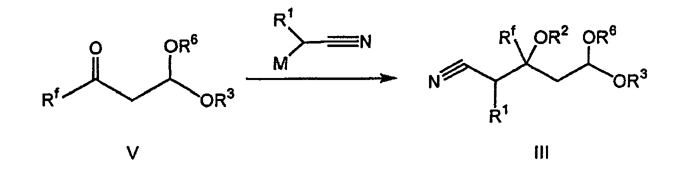 where
M is Li, Na, K, MgY, Mg_{0.5}, CaY, Ca_{0.5}, ZnY, Zn_{0.5}, CdY, Cd_{0.5}, Cu, TiY₃ and
Y is X or I, OR, O-CO-R.

6. Process according to any of Claims 1 to 5, **characterized in that** R² = hydrogen.

7. Process according to any of Claims 1 to 6, **characterized in that** the R_{f} radical is trifluoromethyl.

8. Process according to any of Claims 1 to 7, **characterized in that** the nitrogen compound is an amine.

9. Process according to any of Claims 1 to 8, **characterized in that** the nitrogen compound is ammonia.

## Revendications

1. Procédé de fabrication d'un dérivé de 2-aminopyridine de formule générale II, **caractérisé en ce que** l'on fait réagir un précurseur de nitrile I ou III à chaîne ouverte avec un composé d'azote dans une réaction de cyclisation : 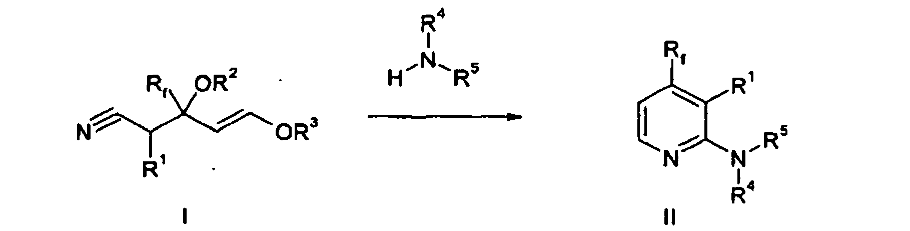 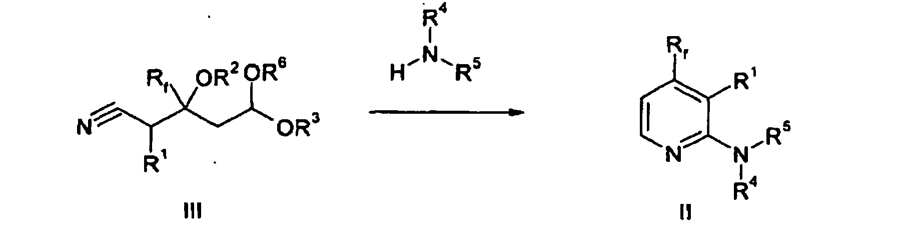 dans laquelle les substituants R¹ à R⁶ et R^{f} ont les significations suivantes :
R_{f} représente CₙH₍₂ₙ₊₁₋ₘ₎Xₘ
X représente les éléments F, Cl, Br, dans lequel pour m > 1, X est identique ou différent
n est un nombre entier positif compris entre 1 et 8
m est un nombre entier positif compris entre 1 et 2n+1
R¹ représente l'hydrogène, un groupement alkyle en C1-C8, aryle en C6-C14, hétéroaryle en C1-C13, COOR, CN, SO₂R, SOR, PO(OR)₂, ces radicaux alkyle, aryle, hétéroaryle, -COOR, -CN, -SO₂R, -SOR et -PO(OR)₂ étant non substitués ou substitués une ou plusieurs fois,
R² représente l' hydrogène ou un groupement protecteur pour des alcools
R³, R⁶ représentent un groupement alkyle en C1-C8, acyle en C1-C8, aryle en C6-C14, R₃-silyle, les radicaux alkyle, acyle et aryle étant non substitués ou substitués une ou plusieurs fois,
R⁴, R⁵ représente l'hydrogène, un groupement alkyle en C1-C8, aryle en C6-C14, hétéroaryle en C1-C13, acyle en C1-C8, -CONR₂, ces radicaux alkyle, aryle, hétéroaryle, acyle et -CONR₂ étant non substitués ou substitués une ou plusieurs fois, ou R⁴ et R⁵ forment conjointement une chaîne alkylène en C4-C8, où un groupement CH₂ peut également être remplacé par NH ou O et où cette chaîne en C4-C8 est non substituée ou substituée une ou plusieurs fois
R représente un groupement alkyle en C1-C8, aryle en C6-C14 ou hétéroaryle en C1-C13.

2. Procédé selon la revendication 1, dans lequel la cyclisation est réalisée à partir de l'énoléther I.

3. Procédé selon la revendication 2, **caractérisé en ce que** l'énoléther I est obtenu à partir de la cétone IV par réaction avec de l'acétonitrile métallé ou un dérivé d'acétonitrile métallé selon le schéma réactionnel suivant : 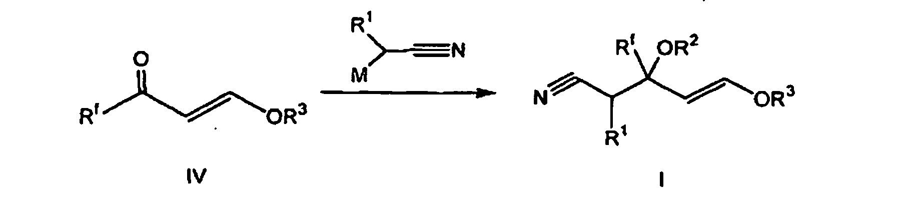 où
M représente Li, Na, K, MgY, Mg_{0,5}, CaY, Ca_{0,5}, ZnY, Zn_{0,5}, CdY, Cd_{0,5}, Cu ou TiY₃ et
Y représente X ou I, OR, O-CO-R.

4. Procédé selon la revendication 1, dans lequel la cyclisation est réalisée à partir de l'acétal III.

5. Procédé selon la revendication 4, **caractérisé en ce que** l'acétal III est obtenu à partir d'une cétone V par réaction avec de l'acétonitrile métallé ou un dérivé d'acétonitrile métallé au sens du schéma réactionnel suivant : 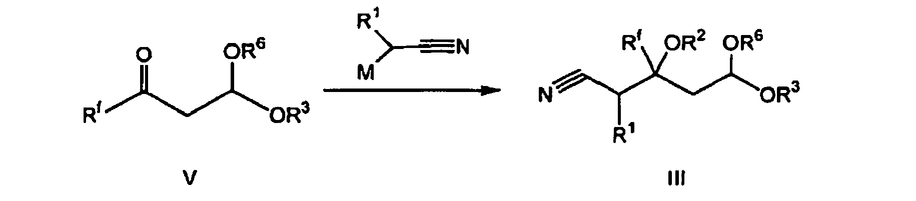 où
M représente Li, Na, K, MgY, Mg_{0,5}, CaY, Ca_{0,5}, ZnY, Zn_{0,5}, CdY, Cd_{0,5}, Cu ou TiY₃ et
Y représente X ou I, OR, O-CO-R.

6. Procédé selon l'une quelconque des revendications 1 à 5, **caractérisé en ce que** R² représente l'hydrogène.

7. Procédé selon l'une quelconque des revendications 1 à 6, **caractérisé en ce que** le radical R_{f} est identique au trifluorométhyle.

8. Procédé selon l'une quelconque des revendications 1 à 7, **caractérisé en ce que** le composé d'azote est une amine.

9. Procédé selon l'une quelconque des revendications 1 à 8, **caractérisé en ce que** le composé d'azote est l'ammoniac.
